# EUROPEAN PATENT APPLICATION

(11) **EP 4 553 158 A1**
(43) Date of publication of application: **14.05.2025**
(21) Application number: 23852009.2
(22) Date of filing: 11.08.2023
(51) Int. Cl.: C12N 15/53, C12N 15/29, C12N 15/82, C12Q 1/68

(54) **HSL PROTEIN, GENE, VECTOR, CELL, COMPOSITION AND USE THEREOF, AND METHOD FOR IMPROVING HERBICIDE RESISTANCE OF CROPS**

(30) Priority: 11.08.2022 CN 202210963026
(71) Applicant: Central China Normal University, Wuhan, Hubei 430079 (CN)
(72) Inventor: YANG, Guangfu, Wuhan, Hubei 430079 (CN); LIN, Hongyan, Wuhan, Hubei 430079 (CN); DONG, Jin, Wuhan, Hubei 430079 (CN)
(74) Representative: reuteler & cie SA
(86) International application number: PCT/CN2023/112677
(87) International publication number: WO 2024/032789

(57) **Abstract**

The invention relates to the field of genetic engineering, and discloses an HSL protein, gene, vector, cell, composition and use thereof, and method for improving herbicide resistance of crops, and particularly discloses an HSL protein, a gene for encoding the HSL protein, a recombinant vector, a transgenic cell, a composition and use thereof in improving herbicide resistance of the crops, and also discloses a method for improving herbicide resistance of the crops. The HSL protein and the encoding gene provided by the invention can be used for improving the resistance of crops to HPPD inhibitors.

## Description

### Cross Reference to Related Applications

The present application claims the benefit of Chinese patent application 202210963026.0, filed on 11/08/2022, the contents of which are incorporated herein by reference.

### Technical Field

The invention relates to the field of genetic engineering, in particular to an HSL protein, gene, vector, cell, composition and use thereof, and method for improving herbicide resistance of crops.

### Background

The gene HIS1 in rice, namely p-hydroxyphenylpyruvate dioxygenase inhibitor sensitive gene 1 (4-hydroxyphenylpyruvate dioxygenase inhibitor sensitive gene No.1), can encode a non-heme ferrous, alpha-ketoglutarate dependent oxygenase, which can hydroxylate a triketone HPPD inhibitor, thereby enabling rice to have resistance to the HPPD inhibitor. Furthermore, a number of homologous genes to HIS1 were found in rice, named HSL (HIS 1-Like). Meanwhile, the HSL gene is also found in other plants, such as barley, wheat, sorghum, maize and Arabidopsis thaliana.

At present, the research on HIS1 and HSL discovers that the protein encoded by HIS1 and the homologous gene HSL thereof can catalyze the triketone HPPD inhibitor, and the inhibition activity of the catalytic product on HPPD is greatly reduced. Since HPPD is an important herbicide target, the important function of HIS1 and HSL in being able to detoxify HPPD inhibitors could be used to develop HPPD-resistant herbicide crops.

There are mainly 13 HPPD inhibitor herbicides currently on the market. Among the more than 20 classes of herbicides worldwide, HPPD inhibitor class herbicides have grown faster in recent years, receiving industry attention, and their low risk of resistance is the cornerstone of the leading growth achieved by this class of products. Based on the better product characteristics of the HPPD inhibitor herbicides, the market of the HPPD inhibitor herbicides still has growth potential in the future.

However, HPPD inhibitor herbicides are used in corn fields and wheat fields in many cases, and are not widely used in commercial crop cultivation areas such as peanuts, soybeans, rice, sorghum, and the like because of crop safety problems. In addition, there are some highly active HPPD inhibitors in the development stage that have not been further developed due to lack of certain crop safety. Herbicide resistant crops, such as glyphosate resistant corn and soybean, have provided enormous benefits to farmers and the environment over the last 20 years.

Therefore, the development of new herbicide-resistant crops, such as crops resistant to HPPD inhibitor herbicides, has important theoretical significance and economic value.

### Disclosure of Invention

The invention aims to overcome the defect that the inhibition activity and the crop safety of the conventional HPPD inhibitor herbicide cannot be compatible.

In order to achieve the above aims, a first aspect of the present invention provides an HSL protein having an amino acid sequence selected from at least one of the following:
(1) a first amino acid sequence derived from the mutation of at least one of the sites 140, 204, 298 and 335 in the amino acid sequence shown in SEQ ID NO: 1;
(2) a protein derived from the first amino acid sequence by substituting, deleting or adding one or at least two amino acids in the first amino acid sequence, or a protein represented by an amino acid sequence with a tag connected to the amino terminal and/or the carboxyl terminal of the first amino acid sequence.

A second aspect of the present invention provides a gene encoding an HSL protein, a nucleotide sequence of the gene is a nucleotide sequence capable of encoding the amino acid sequence of the HSL protein described in the first aspect above.

A third aspect of the present invention provides a recombinant vector, the recombinant vector comprising the gene described in the second aspect above.

A fourth aspect of the present invention provides a transgenic cell, the transgenic cell comprising the gene described in the second aspect above.

A fifth aspect of the present invention provides a composition, the composition comprising the HSL protein described in the first aspect above.

A sixth aspect of the present invention provides the use of at least one of the HSL protein described in the first aspect, the gene described in the second aspect, the recombinant vector described in the third aspect and the transgenic cell described in the fourth aspect in improving the herbicide resistance of crops.

A seventh aspect of the present invention provides a method for improving the herbicide resistance of crops, the method comprises: transferring the recombinant vector described in the third aspect into a target plant, so that the target plant expresses the HSL protein described in the first aspect above to obtain herbicide resistance.

A eighth aspect of the present invention provides a method for improving the herbicide resistance of crops, the method comprises: transferring the recombinant vector in the mutant crops containing the recombinant vector described in the third aspect into a target plant through hybridization, transfer or backcross, so that the target plant expresses the HSL protein described in the first aspect above to obtain the herbicide resistance.

A ninth aspect of the present invention provides a method for improving the herbicide resistance of crops, the method comprises: transforming the HSL gene of a target plant by a CRISPR/Cas gene editing method, so that the target plant expresses the HSL protein described in the first aspect above to obtain the herbicide resistance.

Compared with the prior art, an HSL protein with resistance to the HPPD inhibitor and the encoding gene provided by the invention have at least the following advantages:
By transferring the gene encoding the HSL protein provided by the invention into a target plant, the target plant can acquire resistance to an HPPD inhibitor (herbicide). Therefore, the HSL protein and its encoding gene provided by the invention can be used for cultivating plants with herbicide resistance.

Other features and advantages of the invention will be described in detail in a subsequent section on specific embodiments.

### Drawings

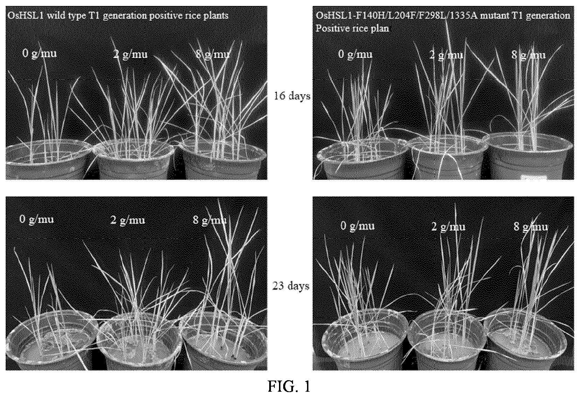

FIG. 1 shows the sensitivity of T1 generation positive rice plants of OsHSL1_F140H/L204F/F298L/I335A mutant and T1 generation positive rice plants of OsHSL1 wild type to the HPPD inhibitor methyl-benquitrione.

### Detailed Description

The endpoints of the ranges and any values disclosed herein are not limited to the precise range or value, and these ranges or values shall be understood to encompass values adjacent to these ranges or values. For numerical ranges, each range between its endpoints and individual point values, and each individual point value can be combined with each other to give one or more new numerical ranges, and such numerical ranges should be construed as specifically disclosed herein.

As mentioned previously, a first aspect of the present invention provides an HSL protein having an amino acid sequence selected from at least one of the following:
(1) a first amino acid sequence derived from the mutation of at least one of the sites 140, 204, 298 and 335 in the amino acid sequence shown in SEQ ID NO: 1;
(2) a protein derived from the first amino acid sequence by substituting, deleting or adding one or at least two amino acids in the first amino acid sequence, or a protein represented by an amino acid sequence with a tag connected to the amino terminal and/or the carboxyl terminal of the first amino acid sequence.

Preferably, the following amino acid sequences are not included in the first amino acid sequence:
(I) an amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into phenylalanine shown in SEQ ID NO: 1.

According to a specific embodiment of the present invention, a amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into phenylalanine shown in SEQ ID NO: 1 named F140H/L204F/F298L/I335F.

Preferably, the first amino acid sequence is an amino acid sequence selected from at least one of the following:
(1a) an amino acid sequence derived from where the isoleucine at site 335 is mutated into phenylalanine shown in SEQ ID NO.1;
(1b) an amino acid sequence derived from where the phenylalanine at site 140 is mutated into histidine, the leucine at site 204 is mutated into phenylalanine, and the isoleucine at site 335 is mutated into phenylalanine shown in SEQ ID NO: 1;
(1c) an amino acid sequence derived from where the leucine at site 204 is mutated into phenylalanine, the phenylalanine at site 298 is mutated into leucine and the isoleucine at site 335 is mutated into phenylalanine shown in SEQ ID NO.1;
(1d) an amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into alanine shown in SEQ ID NO.1;
(1e) an amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into tryptophan shown in SEQ ID NO.1.

Preferably, the first amino acid sequence is at least one of amino acid sequences shown in I335F, F140H/L204F/I335F, L204F/F298L/I335F, F140H/L204F/F298L/I335A, F140H/L204F/F298L/I335W.

According to a specific embodiment of the present invention, a amino acid sequence derived from where the isoleucine at site 335 is mutated to phenylalanine shown as SEQ ID NO.1, is named I335F.

According to a specific embodiment of the present invention, a amino acid sequence derived from where the phenylalanine at site 140 is mutated to histidine, the leucine at site 204 is mutated to phenylalanine and the isoleucine at site 335 is mutated to phenylalanine shown in SEQ ID NO.1, is named F140H/L204F/I335F.

According to a specific embodiment of the present invention, a amino acid sequence derived from where the leucine at site 204 is mutated to phenylalanine, the phenylalanine at site 298 is mutated to leucine and the isoleucine at site 335 is mutated to phenylalanine shown in SEQ ID NO.1, is named L204F/F298L/I335F.

According to a specific embodiment of the present invention, a amino acid sequence derived from where the phenylalanine at site 140 is mutated to histidine, the leucine at site 204 is mutated to phenylalanine, the phenylalanine at site 298 is mutated to leucine, and the isoleucine at position 335 is mutated to alanine shown in SEQ ID NO.1, is named F140H/L204F/F298L/I335A.

According to a specific embodiment of the present invention, a amino acid sequence is derived from where the the phenylalanine at site 140 is mutated to histidine, the leucine at site 204 is mutated to phenylalanine, the phenylalanine at site 298 is mutated to leucine and the isoleucine at site 335 is mutated to tryptophan shown in SEQ ID NO.1, is named F140H/L204F/F298L/I335W.

In the invention, after understanding the scheme of the present invention, the method for obtaining the protein can be easily obtained by those skilled in the art according to the known techniques in the art, for example, in the case of knowing the amino acid sequence of the protein, the protein can be obtained directly by a chemical synthesis method, or the protein can be obtained by obtaining the gene encoding the protein first and then by a biological expression method. For example, the proteins of the present invention can be obtained by methods provided in the Journal of the American Chemical Society2021, (143), 15674-15687 literature by reference to the ratio identification of Enzyme via the Combination of Quantum Mechanics/Molecular Mechanics, Molecular Dynamics, and Structural Biology study.

The proteins provided by the invention may also be modified. Modifications (which do not generally alter primary structure, i.e., do not alter amino acid sequence) include: chemically derivatized forms of the protein, such as acetylation or hydroxylation, in vivo or in vitro. Modified forms also include glycosylation, such as those proteins that result from modification of glycosylation during synthesis and processing of the protein or during further processing steps, which can be accomplished by exposing the protein to an enzyme that performs glycosylation, such as a mammalian glycosylase or deglycosylase. Modified forms also include sequences having phosphorylated amino acid residues (e.g., phosphotyrosine, phosphoserine, phosphothreonine).

In the present invention, for the convenience of purification, the protein can be modified by adding tags that are common in the field, and exemplarily, the protein can be obtained by attaching a common purification tag (e.g., at least one of GST, Poly-His, FLAG, His-SUMO, and c-myc) shown in Table 1 to the amino terminus and/or the carboxy terminus of the protein. The tag does not influence the activity of the protein provided by the invention, and whether the tag is added or not can be selected according to requirements in the practical application process.

**Table 1**

| Tag | Number of residues | Amino acid sequence |
|---|---|---|
| GST | 218 | MSPILGYWKIKGLVQPTRLLLEYLEEKYEEHLYERD EGDKWRNKKFELGLEFPNLPYYIDGDVKLTQSMAII RYIADKHNMLGGCPKERAEISMLEGAVLDIRYGVSR IAYSKDFETLKVDFLSKLPEMLKMFEDRLCHKTYLN GDHVTHPDFMLYDALDVVLYMDPMCLDAFPKLVC FKKRIEAIPQIDKYLKSSKYIAWPLQGWQATFGGGD HPPK |
| Poly-His | 2-12 (generally 6) | HHHHHHHH |
| FLAG | 8 | DYKDDDDK |
| His-SUMO | 111 | MGSSHHHHHHGSSMSDSEVNQEAKPEVKPEVKPET HINLKVSDGSSEIFFKIKKTTPLRRLMEAFAKRQGKE MDSLRFLYDGIRIQADQTPEDLDMEDNDIIEAHREQI GG |
| c-myc | 10 | EQKLISEEDL |

As mentioned previously, a second aspect of the present invention provides a gene encoding an HSL protein, a nucleotide sequence of the gene is a nucleotide sequence capable of encoding the amino acid sequence of the HSL protein described in the first aspect above.

Preferably, the nucleotide sequence of the gene is a nucleotide sequence selected from at least one of the following:
(1) a nucleotide sequence shown as SEQ ID NO. 11;
(2) a nucleotide sequence having at least 90% identity, preferably at least 91% identity, at least 92% identity, at least 93% identity, at least 94% identity, at least 95% identity, at least 96% identity, at least 97% identity, at least 98% identity, or at least 99% identity to SEQ ID NO. 11.

In the present invention, it is known in the field that of the 20 different amino acids that make up proteins, except for Met (ATG) or Trp (TGG), which are each encoded by a single codon, the other 18 amino acids are encoded by 2 to 6 codons, respectively, (Sambrook et al, molecular cloning, Cold spring harbor laboratory Press, New York, U.S. Pat. No., second edition, 1989, see appendix D page 950). That is, due to the degeneracy of genetic code, most of the codons that determine an amino acid are more than one, and the substitution of the third nucleotide in a triplet codon does not change the composition of the amino acid, so the nucleotide sequences of genes encoding the same protein can be different.

The person in the field, based on the known codon table, derives from the amino acid sequence SEQ ID NO: 1 and the specific mutation mode of the protein provided by the invention, the nucleotide sequences of the gene capable of encoding them can be deduced completely, and the nucleotide sequences can be obtained by a biological method (such as a PCR method and a mutation method) or a chemical synthesis method, so that the partial nucleotide sequences all should be included in the scope of the invention.

Similarly, the nucleotide sequence SEQ ID NO: 11 and the specific mutation mode of the protein provided by the invention, can also be performed by methods known in the art, such as Sambrook et al (molecular cloning, Cold spring harbor laboratory Press, New York, U.S. second edition, 1989), by modifying the amino acid sequence of SEQ ID NO: 11, obtaining the amino acid sequence provided by the invention.

In the present invention, On the basis of knowing the above protein and nucleotide sequence of SEQ ID NO: 11, those skilled in the field can obtain the gene encoding the protein provided by the present invention by means known in the field. Exemplarily, site-directed mutagenesis can be carried out on the basis of SEQ ID NO: 11, including but not limited to ZFN site-directed mutagenesis, TALEN site-directed mutagenesis and/or genome site-directed mutagenesis such as CRISPR-Cas9.

In the present invention, as described in the first aspect, the 5 'end and/or 3' end of the nucleotide sequence provided by the present invention can also be linked with a coding sequence of a common purification tag shown in Table 1, respectively.

The nucleotide sequence provided by the present invention can be obtained by a Polymerase Chain Reaction (PCR) amplification method, a recombination method, or an artificial synthesis method. Exemplarily, one skilled in the field can easily obtain templates and primers and obtain the nucleotide sequence by PCR amplification. After obtaining the nucleotide sequence, the amino acid sequence can be obtained in large quantities by recombinant methods. The nucleotide sequence obtained is usually cloned into a vector, then transferred into a genetic engineering bacterium, and then separated from a propagated host cell by a conventional method to obtain the nucleotide sequence. In addition, the nucleotide sequence can be synthesized by artificial chemical synthesis methods known in the field.

As mentioned previously, a third aspect of the present invention provides a recombinant vector, the recombinant vector comprising the gene described in the second aspect above.

As the "vector" used in the recombinant vector of the present invention, various vectors known in the field, such as various commercially available plasmids, cosmids, phages, retroviruses, and the like, can be used, and they can be selected according to the particular circumstances, exemplarily, it can be pGWC, pB2GW7.0, or pET-28a, etc. The recombinant vector can be constructed by using various endonucleases with cutting sites at the multiple cloning sites of the vector to obtain linear plasmids, which can be connected with gene segments cut by the same endonucleases to obtain the recombinant plasmids.

As mentioned previously, a fourth aspect of the present invention provides a transgenic cell, the transgenic cell comprising the gene described in the second aspect above.

Preferably, the transgenic cell is a prokaryotic cell.

In the present invention, the recombinant vector can be transformed, transduced or transfected into a host cell by a method known in the field, such as chemical transformation by calcium chloride method, high-voltage shock transformation, preferably shock transformation. The host cell may be prokaryotic or eukaryotic and may be selected according to the actual circumstances. The cell can be DH5 alpha strain, Agrobacterium strain GV3101, etc.

As mentioned previously, a fifth aspect of the present invention provides a composition, the composition comprising the HSL protein described in the first aspect above.

The composition provided by the present invention comprises the HSL protein described in the first aspect of the present invention as an active ingredient, and the content of the protein is 50 to 90 wt% based on the total weight of the composition. The composition can further comprise conventional solvents known to those skilled in the field (such as glycerol, saccharides, and protease inhibitors) and the like.

As mentioned previously, a sixth aspect of the present invention provides the use of at least one of the HSL protein described in the first aspect, the gene described in the second aspect, the recombinant vector described in the third aspect, and the transgenic cell described in the fourth aspect in improving the herbicide resistance of crops.

Preferably, the herbicide is an HPPD inhibitor.

Preferably, the herbicide is at least one of a triketone compound, a pyrazole compound, an isoxazole compound, a diketone nitrile compound and a heterocyclic amide compound.

More preferably, the herbicide is at least one of mesotrione, tembotrione, tefuryltrione, benzobicyclon technical, benquitrione, methyl-benquitrione, compound A, pyraquinate technical and topramezone, and the molecular structural formula of the herbicide is as shown in the following formula:

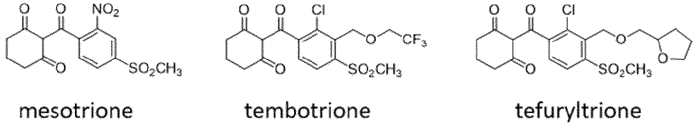

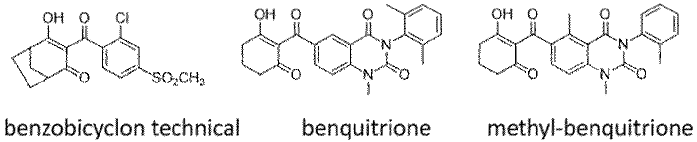

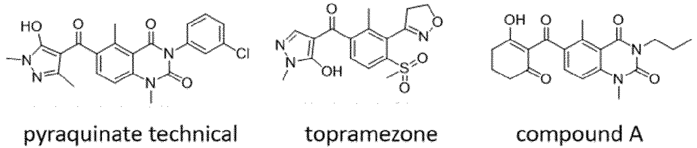

As mentioned previously, a seventh aspect of the present invention provides a method for improving the herbicide resistance of crops, the method comprises: transferring the recombinant vector described in the third aspect into a target plant, so that the target plant expresses the HSL protein described in the first aspect above to obtain herbicide resistance.

As mentioned previously, a eighth aspect of the present invention provides a method for improving the herbicide resistance in of crops, the method comprises: transferring the recombinant vector in the mutant crops containing the recombinant vector described in the third aspect into a target plant through hybridization, transfer or backcross, so that the target plant expresses the HSL protein described in the first aspect above to obtain the herbicide resistance.

As mentioned previously, a ninth aspect of the present invention provides a method for improving the herbicide resistance of crops, the method comprises: transforming the HSL gene of a target plant by CRISPR/Cas gene editing method, so that the target plant expresses the HSL protein described in the first aspect above to obtain the herbicide resistance.

In the present invention, transferring the recombinant vector into a target plant specifically refers to: a nucleotide sequence of the protein with herbicide resistance provided by the invention is transferred to the target plant by a transgenic method to ensure that the target plant can obtain the resistance to the HPPD inhibitor herbicides; the nucleotide sequence of the protein provided by the invention can also be transferred into the target plant by methods such as hybridization, transfer, backcross, etc, so that the target plant can obtain the resistance to the HPPD inhibitor herbicides; in addition, the HSL gene of the target plant can be directly modified by CRISPR/Cas and other gene editing technologies, so that the target plant can obtain the resistance to the HPPD inhibitor herbicides. More specifically, the protein can be used as a parent material, and is hybridized with other excellent plant varieties and further backcrossed, so that the herbicide-resistant character is further transferred into other target plant varieties.

The present invention has no special requirements for the operation process and specific parameters of the applied transgenic method, which can be performed in a manner known in the field, the transgenic method includes a direct or indirect conversion method, and the direct conversion method includes chemical substance induction method, liposome method, gene gun method, electroporation method and microinjection method, etc. For example, the transgenic method of the present invention can be carried out by referring to the method provided in A protocol for Agrobacterium-mediated transformation in rice Nature protocols2006, (1), 2796-2802 documents.

In the present invention, the term "plant" has the broadest meaning, and examples of plants include, but are not limited to, vascular plants, vegetables, foodstuffs, flowers, arbor, herbaceous plants, shrubs, grasses, vines, ferns, moss, fungi, algae, and the like, and clones and plant parts used for asexual propagation (e.g.,cuttings, layering, shoots, rhizomes, underground stems, clumps, root necks, bulbs, corms, tubers, rhizomes, plants/tissues produced in tissue culture, etc.). The term "plant" further encompasses whole plants, plant parents and progeny, and plant parts, including seeds, branch, stems, leaves, roots (including tubers), flowers, florets, fruits, stalks, inflorescence stems, stamens, anthers, stigma, style, ovaries, petals, sepals, carpels, root tips, root crowns, root hairs, leaf hairs, hair, pollen grains, microspores, cotyledons, hypocotyls, epicotyls, xylem, phloem, parenchyma, endosperm, companion cells, guard cells, and any other known organ, tissue and cell, and tissue and organ of plants. The term "plant" also encompasses plant cells, suspension cultures, callus tissue, embryos, meristematic regions, gametophytes, sporophytes, pollen, and microspores. Wherein, all mentioned above include the target gene/nucleic acid provided by the invention.

Plants which are particularly suitable for use in the method of the invention include all plants belonging to the superfamily of plants (viridiplantaae), in particular monocotyledonous and dicotyledonous plants, including food crops, feed or forage legumes, ornamentals, arbor or shrubs.

According to a preferred embodiment of the invention, the plants are crop plants. Examples of crop plants include, rice, wheat, corn, sorghum, soybean, sunflower, oilseed rape, alfalfa, cotton, tomato, potato or tobacco. More preferably, the crop plant is a cereal, such as rice, wheat, maize, sorghum, barley, millet, rye or oats.

The invention has the advantages that the protein, the gene, the recombinant vector, the transgenic cell, the composition and the plant which can replace the prior art and can enable the crops to have herbicide resistance are obtained, the application and the method of the crops with herbicide resistance are obtained, the crop variety with herbicide resistance can be obtained by a transgenic or non-transgenic method, and the weed control in the cultivated paddy field and the cultivated upland field can be more efficiently carried out, so that the yield and the yield stability of the crops can be improved.

In the following examples, the room temperature means 25±2 °C unless otherwise specified.

In the following examples, the experimental methods used are all those conventionally known in the field unless otherwise specified.

In the following examples, the materials, reagents, etc involved are commercially available unless otherwise specified.
Agarose gel recovery kits were purchased from TIANGEN Company;
Restriction enzyme and its matching solution were purchased from Takara Company;
Connection kits were purchased from New England BioLabs.

### Example 1

### Synthesis of coding genes and nucleotide sequences

The following coding genes and nucleotide sequences were synthesized from the nucleotide sequences provided by Wuhan Kerui bioengineering, Inc. according to the present invention.

### (1) SEQ ID NO: 11

The coding gene of the protein shown in Table 2 and the nucleotide sequence shown in SEQ ID NO: 11 were synthesized according to the nucleotide sequence shown in SEQ ID NO: 11.

**Table 2**

| Protein | Coding gene | Mutant site codons |
|---|---|---|
| SEQ ID NO: 1 | *WT (wild type)* | / |
| I335F | *I335F* | TTT |
| F140H/L204F/I335F | *F140H*/*L204F*/*I335F* | CAT/TTT |
| L204F/F298L/1335F | *L204F*/*F298L*/*I335F* | TTT/CTG/TTT |
| F140H/L204F/F298L/I335A | *F140H*/*L204F*/*F298L*/*I335A* | CAT/TTT/CTG/GCG |
| F140H/L204F/F298L/I335W | *F140H*/*L204F*/*F298L*/*I335W* | CAT/TTT/CTG/TGG |

### Example 2

### Construction of expression vectors

### (1) Amplification of the target gene

The nucleotide sequence of SEQ ID NO: 11 was used as the template, the wild type and mutant gene sequences of OsHSL1 as shown in Table 2 were obtained by PCR reaction and connected to the corresponding vectors.

The PCR reaction system and PCR program set-up are shown in Table 3 and Table4 below, respectively:

**Table 3**

| PCR reaction System composition | Addition amount (volume) |
|---|---|
| 10×PCR buffer fluid | 5µL |
| 5×dNTP (10mM) | 1µL |
| Template | 1µL |
| Forward primer | 1.0µL |
| Direction primer | 1.0µL |
| PfuDNA polymerase | 0.5µL |
| dd H₂O | To a total volume of 50µL |

**Table 4**

| Step | Temperature (°C) | Time(s) |
|---|---|---|
| 1 | 95 | 240 |
| 2 | 95 | 45 |
| 3 | 55 | 45 |
| 4 | 72 | 400 |
| 5 | 72 | 600 |
| 6 | 4 | Preservation |

The extension time at 72 °C depends on the specific fragment length and the efficiency of the enzyme used. The above procedure was repeated for 25 cycles from step 2 to step 4.

### (2) Nucleic acid agarose gel electrophoresis and gel recovery PCR product

a. Weighing 1g of agarose in a conical flask, adding 100mL of 1×TAE solution (preparing 50 times of storage solution, preparation method: weighing 242g of Tris and 18.6g of EDTA in a 1L beaker, adding about 800mL of deionized water, stirring, adding 57.1mL of glacial acetic acid, fully dissolving, adjusting the pH to 8.3 by NaOH, adding deionized water to constant volume of 1L, preserving at room temperature, and the same below), heating in a microwave oven, adding 5 µL of ethidium bromide solution after the agarose particles were completely dissolved and cooled to be not hot, mixing uniformly, pouring into the glue tank where the comb has been placed, and letting it cool and solidified;
b. the comb was pulled out before use, the gel was put into an electrophoresis tank, and a proper amount of 1×TAE solution was added; adding the sample into the hole, covering the cover to start electrophoresis, and stopping electrophoresis when bromophenol blue moves to a position far away from the bottom 1/3;
c. cutting the target strip, putting the cut target strip into a clean 1.5 mL EP tube and recovering glue; the kit for gel recovery was purchased from TIANGEN company (Item No.DP 209); operating according to the amount of 100mg of glue corresponding to 300 muL of sol solution PN (provided by a kit), and incubating in a water bath at 50 °C, and continuously turning upside down and uniformly mixing to help the glue to dissolve;
d. adding the solution in the step c into a pretreated adsorption column (provided by a kit), standing at room temperature for 5min to fully combine, and centrifuging at 12000rpm for 1 min;
e. pouring out the liquid in the collecting pipe, and repeating the step d once to increase the recovery rate of the product;
f. adding 500 µL of rinsing solution PW (provided by kit) to the purification column, and centrifuging at 12000rpm for 1 min; repeating the operation steps once;
g. centrifuging the adsorption column at 13000rpm for 2min to remove PW solution as much as possible;
h. putting the purification column into a new EP tube, heating at 65 °C, standing for 10min, and volatilizing PW solution;
i. dropwise adding 50 µL of elution solution (provided by a kit) to the middle position of the adsorption column, placing the adsorption column on a 65 °C heating table for 2min, and centrifuging at 13000rpm for 2min to elute DNA; repeating the operation steps once;
j. finally, the recovered PCR product was stored at-20 °C.

### (3) Enzyme digestion reaction

After electrophoresis verification, DNA with correct size fragments was obtained, and double enzyme digestion was carried out on the vector and the PCR product (enzyme digestion solution was purchased together with restriction enzyme). The enzyme digestion system is shown in Table 5:

**Table 5**

| Components | Volume (µL) |
|---|---|
| PCR product/vector | 43 |
| 10×Cutsmart solution | 5 |
| BamHI | 1 |
| XhoI | 1 |

After Incubation at 37 °C for 3h, electrophoresis was carried out and recovered by gel recovery kit; in order to improve the efficiency of vector cutting, 1.5µL restriction endonuclease was added and the cutting time was prolonged for 5 h.

### (4) Connection reaction

The recovered gene fragments were connected by using the following system, and the connection reagent Solution1 (containing T4DNA ligase and buffer) was purchased from New England Biolabs (Item # M0202S), followed by incubation at 16 °C for 5 hours or more. The connection system is shown in Table 6:

**Table 6**

| Composition | Volume (µL) |
|---|---|
| Solution 1 | 8 |
| target fragment | 7 |
| vector | 1 |

### (5) Transformation

After connection, the connection products were transformed into competent cells of Escherichia coli JM109 (purchased from Promega, product No. ST 1105) and cultured as follows:
a. taking out a tube of competent cells, and placing the tube of competent cells on ice to melt for 10 min;
b. adding the connection product into the competent cells, mixing uniformly, and standing on ice for 30 min;
c. heat shock at 42 °C for 90s, then placing on ice for 2 min;
d. 200µL of Luria-Bertani (LB) medium (containing no antibiotics) was added to the tube, and cultured at 37 °C and 220rpm for 30 min; the solid LB plate with corresponding resistance was preheated at room temperature;
e. taking out the bacterial liquid, uniformly coating the bacterial liquid on a flat plate, and culturing for 16h in a 37 °C thermostat.

### (6) Sequencing

Selecting some monoclonals from the monoclonals cultured on the plate for 16h, transferring the monoclonals to a clean plate, culturing the monoclonals at 37 °C for 5h, and then sending the monoclonals to Wuhan King Kerui bioengineering limited company for sequencing; and after a sequencing result is fed back, performing sequence comparison to confirm correct cloning.

### Example 3

### Expression and purification of target proteins

### (1) Expression

Firstly, carrying out small extraction on the protein, searching for the optimal expression condition and purification strategy, and then carrying out large-scale extraction after determining the conditions. Protein expression was performed in E.coli BL21 (DE 3) cells (purchased from Kangsheng Life technologies, Inc., product number KTSM 109) as follows:
a. selecting a single clone on a plate cultured for 16h, adding the single clone into 100mL of LB culture medium containing the required resistance antibiotics, and culturing for 5h at 37 °C and 220rpm, namely observing obvious turbidity;
b. The vials of bacteria in step a were expanded to the larger vials of LB medium (containing antibiotics) at the volume ratio of 1:100, and cultured at 37°C and 220rpm for 3h; when the OD600 value reached 0.7h, the temperature was reduced to 20 °C, and then 0.2mM isopropyl-beta-D-thiogalactoside (IPTG) was added for induction for 14 h;
c. After centrifugation at 4 °C and 4000rpm for 10min, the bacteria were collected and used for protein purification.

### (2) Purification

a. The collected escherichia coli was suspended with cell lysis solution, and the volume ratio between the cell lysis solution and the LB culture solution for collecting the escherichia coli was 30 mL: 1L; the mixture were stirred on a magnetic stirrer for 20min to mix the cells uniformly, during the mixing, 1mM of the serine protease inhibitor phenylmethylsulfonyl fluoride (PMSF), 40g/mL of lysozyme for cracking bacterial cell walls, 1g/mL of deoxyribonuclease I and 10mM of a cofactor MgCl₂ were added; then ultrasonic crushing;
b. After ultrasonic was completed, the supernatant was centrifuged at 13000rpm and 4 °C for 1h, and the supernatant was collected for affinity chromatography; protein purification was carried out in a thermostatic chamber at 4 °C;
c. Firstly, the supernatant after centrifugation was poured into the treated Ni column for full combination; then washing with solutions containing different concentrations of imidazole (10mM, 30mM, 50mM) to remove the impurity proteins; finally the target protein was eluted with imidazole solution containing a high concentration (250mM); then the result were analyzed by SDS-PAGE;
d. The protein after affinity chromatography was diluted and onto the ion exchange column, after sample loading, linear gradient (NaCl from 0 to 500 mM) was eluted with a buffered solution containing NaCl (Tris, pH 7.0), which was completed with the help of a protein purifier; the results were analyzed by SDS-PAGE, and the proteins with good properties and purity were combined and concentrated for the next step of molecular sieve chromatography;
e. Before sample loading , the molecular sieve chromatography should be centrifuged at 13000rpm for 5min to remove part of precipitated protein and remove impurities, and then the sample was sent to the sample loading ring with a syringe; then the protein was eluted with an elution buffer solution (100mM NaCl, Tris ,pH 7.0), and collecting 0.5mL of sample in each tube; SDS-PAGE was used to detect and analyze the proteins, and the proteins that needed to be preserved were combined, and then subpackaging and freezing; the protein for activity measurement was stored by adding glycerol with a final concentration of 30 vol%.

### Test example 1

### Study of the catalytic Activity of HPPD inhibitors

Testing the speed and the degree of catalysis of each protein on the HPPD inhibitor by using a High Performance Liquid Chromatography (HPLC) method on the premise of ensuring that the concentrations of each protein and the HPPD inhibitor are the same.

The specific test method comprises the following steps:
a. preparing a buffer solution, a substrate and a cofactor:
   detection of active HEPES buffer: a storage solution with a concentration of 1M was prepared first, its pH value was adjusted to 7.0 with sodium hydroxide, diluted to 20mM before use, and filtered with 0.22 µm filter membrane;
   formulation of the substrate (i.e. HPPD inhibitor): DMSO was used to prepare a 10mM storage solution, which was diluted with a active buffer.before use;
   Preparation of sodium ascorbate: a concentration of 20mM was formulated with deionized water. Stored at-80°C;
   Preparation of ferrous sulfate: a concentration of 1mM was formulated with deionized water. Stored at-80°C;
   Preparation of alpha-ketoglutaric acid: a concentration of 50mM was formulated with deionized water. Stored at-80°C;
b. when in test, firstly, the active buffer (with the final concentration of 20mM), the substrate (with the final concentration of 50µM), the sodium ascorbate (with the final concentration of 2mM), the ferrous sulfate (with the final concentration of 100µM) and the alpha-ketoglutaric acid (with the final concentration of 1mM) were mixed, and finally the enzyme (with the final concentration of 0.5mg/mL) was added, the total volume was 200µL, and the mixture was reacted at 30 °C for 10 hours;
c. The reaction was terminated by adding 20µL of 10 vol % trifluoroacetic acid, centrifuging at 12000rpm for 10min, and taking 180µL and adding it into the sample bottle for detection;
d. when HPLC was used to detect the sample, the loading volume was 60µL, the absorption peak area of the remaining substrate at 286nm was measured, and the amount of the substrate consumed in the different reactions was calculated according to the standard curve of the substrate (i.e., control), so as to evaluate the catalytic activity of each protein. Each sample was replicated three times.
e. Determination of the standard curve of substrate (HPPD inhibitor): using the same reaction system as above (but without the addition of the enzyme), different substrate concentrations, e.g., 0.1µM, 0.5µM, 1µM, 10µM, 50µM, were set simultaneously to obtain the relationship between substrate concentration and peak area.

Control: refers to the addition of inhibitors to the reaction system, but not the addition of enzymes, which is regarded as the substrate surplus when the inhibitor is not catalyzed.

Sample: in the enzyme reaction system, the same volume and the same concentration (50µM) of the inhibitor was added, and the catalytic efficiency of the enzyme to the inhibitor was observed. Catalytic efficiency = (1- (peak area of substrate in sample)/(peak area of substrate in control)) ×100%.

In this test example, 2 representative HPPD inhibitor herbicides were tested:
Triketones: mesotrione, tembotrione, tefuryltrione, benzobicyclon technical, methyl-benquitrione and compound A;
Pyrazoles: pyraquinate technical and topramezone, the catalytic results of rice HSL1 (i.e. OsHSL 1)) mutant protein on the above HPPD inhibitor were respectively tested, and the specific results were shown in Table 7, and the time dependence of OsHSL1 wild-type protein and OsHSL1 mutant protein (OsHSL 1_I335F) on the degradation degree of the topramezone was tested, and the specific results were shown in Table 8.

### Test example 2

### Enzyme kinetic parameter study of HPPD inhibitors

High Performance Liquid Chromatography (HPLC) was used to test OsHIS1 wild-type protein (the amino acid sequence of OsHIS 1 wild-type is shown as SEQ ID NO 2, and the corresponding nucleotide sequence is shown as SEQ ID NO 21), the enzyme kinetic parameters of OsHSL1 wild-type protein and some OsHSL1_mutant proteins with higher reactivity and HPPD inhibitors, and the specific results are shown in Table 9-Table 12. The specific test method comprises the following steps:
a. preparing a buffer solution, a substrate and a cofactor:
   detection of active HEPES buffer: a storage solution with a concentration of 1M was prepared first, its pH value was adjusted to 7.0 with sodium hydroxide, diluted to 20mM before use, and filtered with 0.22µm filter membrane;
   formulation of the substrate (i.e. HPPD inhibitor): DMSO was used to prepare a 10mM storage solution, which was diluted with a active buffer.before use;
   Preparation of sodium ascorbate: a concentration of 20mM was formulated with deionized water. Stored at-80°C;
   Preparation of ferrous sulfate: a concentration of 1mM was formulated with deionized water. Stored at-80°C;
   Preparation of alpha-ketoglutaric acid: a concentration of 50mM was formulated with deionized water. Stored at-80°C;
b. when in test, firstly, the active buffer (with a final concentration of 20mM), the substrate (with a series of concentrations), the sodium ascorbate (with the final concentration of 2mM), the ferrous sulfate (with the final concentration of 100µM) and alpha-ketoglutaric acid (with the final concentration of 1mM) were mixed, and finally, the enzyme (with a proper concentration) was added, the total volume was 200µL, and the mixture was reacted at 30 °C for about 20min, and the substrate consumption was controlled not to exceed 10%;
c. The reaction was terminated by adding 20µL of 10 vol % trifluoroacetic acid, centrifuging at 12000rpm for 10min, and taking 180µL and adding it into the sample bottle for detection;
d. when HPLC was used to detect the sample, the loading volume was 60µL, the absorption peak area of the remaining substrate at 286nm was measured, and the amount of substrate consumed (reaction rate of enzyme) in the different reactions was calculated according to the standard curve of the substrate (i.e., control), and the enzyme kinetic parameters were obtained by nonlinear fitting the Michaelis-Menten equation using originPro86 software, and the specific results are shown in Table 9 to Table 12. Each sample was replicated three times.
e. Determination of the standard curve of substrate (HPPD inhibitor): using the same reaction system as above (but without the addition of the enzyme), different substrate concentrations, e.g., 0.1µM, 0.5µM, 1µM, 10µM, 50µM, etc., were set simultaneously to obtain the relationship between substrate concentration and peak area.

### Test example 3

### Herbicide resistance test of OsHSL1_F140H/L204F/F298L/I335A mutant over-expression in rice

Successfully overexpressed OsHSL1_F140H/L204F/F298L/I335A mutants and OsHSL1 wild type T0 generation tissue culture seedlings were selected, cultivated and harvested, and then sown to offspring population T1 generation rice plants, and positive over-expression rice plants were identified.

OsHSL1_F140H/L204F/F298L/I335A mutant T1 generation positive rice plants and OsHSL1 wild-type T1 generation positive rice plants at 3 weeks of seedling age were treated with different concentrations of methyl-benquitrione aqueous solution on the Stem leaves, and OsHSL1_wild-type T1 generation positive rice plants as a control. The resistance of mutant OsHSL1_F140H/L204F/F298L/I335A to methyl-benquitrione was tested, and the specific results were shown in Figure 1.

**Table 7: catalysis results of OsHSL1 wild-type protein and OsHSL1 mutant protein on different HPPD inhibitors**

| | Catalytic efficiency (%) | | | | | |
|---|---|---|---|---|---|---|
| | Mesotrion e | benzobicy clon technical | tefuryltrio tembotrion ne e | methyl-benquitrio ne | Compoun dA | topramezo pyraquinat ne e technical |
| OsHSL1 wild type | 40 | 46 | 100 89 | 0 | 0 | 100 17 |
| OsHSL1_I335F | 15 | 44 | 98 62 | 0 | 0 | 100 24 |
| OsHSL1 F140H/L204F/1335F | 89 | 100 | 100 36 | 23 | 35 | 40 2 |
| OsHSL1 L204F/F298L/1335F | 56 | 100 | 94 0 | 35 | 47 | 1 52 |
| OsHSL1 F140H/L204F/F298L/I335A | 90 | 100 | 85 71 | 95 | 97 | 4 40 |
| OsHSL1 F140H/L204F/F298L/1335W | 88 | 100 | 70 55 | 90 | 98 | 0 17 |

| | | | | | | |
|---|---|---|---|---|---|---|
| Note: the greater the catalytic efficiency, the better the degradation effect. The reaction conditions in the table are: the reaction temperature is 30°C, the reaction time is 10h, the substrate (HPPD inhibitor) concentration is 50µM, and the protein concentration is 0.5mg/mL. | | | | | | |

**Table 8: time-dependent results of OsHSL1 wild-type protein and OsHSL1_I335F mutant protein on topramezone degradation degree**

| | Catalytic efficiency (%) | | | | |
|---|---|---|---|---|---|
| | 30min | 60min | 120min | 300min | 600min |
| OsHSL1 wild type | 79 | 86 | 93 | 98 | 100 |
| OsHSL1 I335F | 97 | 100 | 100 | 100 | 100 |

**Table 9: enzyme kinetic parameters for the reaction of wild type protein with mutant protein and mesotrione**

| | *K*ₘ/ µM | *K*_{cat}/s⁻¹ | *K_{cat}lKₘ* |
|---|---|---|---|
| OsHIS1 wild type | 5.5811±0.3314 | 0.05957±-0.00197 | 1.07×10⁻² |
| OsHSL1 wild type | 27.6770±2.8768 | 0.0002±0.00004 | 7.38×10⁻⁶ |
| OsHSL1 F140H/L204F/F298L/I335A | 23.4659±2.2490 | 0.0083±0.0006 | 3.54×10⁻⁴ |

**Table 10: enzyme kinetic parameters for the reaction of wild type protein with mutant protein and methyl-benquitrione**

| | *K*ₘ/ µM | *K*_{cat}/s⁻¹ | *K_{cat}lKₘ* |
|---|---|---|---|
| OsHIS1 wild type | 16.2456+0.9309 | 0.0106+0.0004 | 6.51×10⁻⁴ |
| OsHSL1 wild type | / | / | / |
| OsHSL1 F140H/L204F/F298L/I335A | 22.6001+1.6172 | 0.0106+0.0004 | 4.69×10-⁴ |

| | | | |
|---|---|---|---|
| Note: /- indicates that the enzyme reactivity is too low to be measured. | | | |

**Table 11: enzyme kinetic parameters for wild-type protein reaction with mutant protein and topramezone reaction**

| | *Kₘ*/µM | *K*_{cat}/s⁻¹ | *K_{cat}lKₘ* |
|---|---|---|---|
| OsHIS1 wild type | / | / | / |
| OsHSL1 wild type | 44.0732±4.9393 | 0.0011±0.00003 | 2.53×10⁻⁵ |
| OsHSL1 I335F | 48.4211+1.5049 | 0.0057+0.00016 | 1.19×10⁻⁴ |

| | | | |
|---|---|---|---|
| Note: /- indicates that the enzyme reactivity is too low to be measured. | | | |

**Table 12: enzyme kinetic parameters for the reaction of wild-type protein with mutant protein and pyraquinate technical**

| | *K*ₘ/µM | *K*_{cat}/s⁻¹ | *K_{cat}lKₘ* |
|---|---|---|---|
| OsHIS1 wild type | / | / | / |
| OsHSL1 wild type | / | / | / |
| OsHSL1_ L204F/F298L/1335A | 176.9748±8.4079 | 0.00135±0.00006 | 7.61×10⁻⁶ |

| | | | |
|---|---|---|---|
| Note: /- indicates that the enzyme reactivity is too low to be measured. | | | |

From the results in Table 7, it can be seen that I335 is a key amino acid for the enzyme to react. For the triketone HPPD inhibitor, the OsHSL1 mutant containing the I335 mutation has better degradation effect on the triketone HPPD inhibitor, wherein the degradation effect of the four-point mutant OsHSL1_H140F/L204F/F298L/I335A and the degradation effect of the OsHSL1_H140F/L204F/F298L/I335W are optimal; for pyrazole HPPD inhibitors, the OsHSL1 mutant containing I335 mutation has an increased degradation effect on the pyrazole HPPD inhibitors, and particularly, the single-point mutant OsHSL1_I335F can completely degrade topramezone defense like the wild type OsHSL 1.

From the results in Table 8, it can be seen that the single-point mutant OsHSL1_ I335F has a significantly better effect on topramezone degredation than OsHSL1 wild type.

From the results in Table 9 to Table 12, it can be seen that the four-point mutant OsHSL1_H140F/L204F/F298L/I335A containing the I335 mutation has better affinity and catalytic efficiency for the triketone HPPD inhibitors (mesotrione, benzobicyclon technical and methyl-benquitrione) than the OsHSL1 wild type, and is close to the OsHIS1 wild type; for pyrazole HPPD inhibitors (topramezone and pyraquinate technical), the catalytic efficiency of a single-point mutant OsHSL1_I335F of I335 to topramezone is obviously superior to that of OsHSL1 wild type, and the affinity and the catalytic efficiency of a three-point mutant OsHSL1_L204F/F298L/I335A containing 1335 to pyraquinate technical are superior to that of OsHIS1 wild type and OsHSL1 wild type.

FIG. 1 is a graph showing the sensitivity of OsHSL1_F140H/L204F/F298L/I335A mutant T1 generation positive rice plants and OsHSL1 wild type T1 generation positive rice plants to the HPPD inhibitor methyl-benquitrione. As can be seen from figure 1, under the condition that the spraying concentration of the methyl-benquitrione is 2 g/mu and 8 g/mu, after 16 days of treatment, the OsHSL1 wild type T1 generation positive rice plants almost all have albinism symptoms; albino symptoms become very obvious after 23 days of treatment, while the OsHSL1_F140H/L204F/F298L/I335A mutant T1 generation positive rice plants basically keep normal green, which indicates that the OsHSL1_F140H/L204F/F298L/I335A mutant can improve the resistance of the rice plants to the triketone HPPD inhibitor methyl-benquitrione.

According to the test results, the HSL protein provided by the invention has a remarkable degradation effect on HPPD herbicides. And the OsHSL1_ F140H/L204F/F298L/I335A mutant over-expression rice plants have obvious resistance to methyl-benquitrione. Therefore, the HSL protein provided by the invention can be used for improving the herbicide resistance of crops.

The preferred embodiments of the present invention have been described above in detail, but the present invention is not limited thereto. Within the scope of the technical idea of the invention, many simple modifications can be made to the technical solution of the invention, including various technical features being combined in any other suitable way, and these simple modifications and combinations should also be regarded as the disclosure of the invention, and all fall within the scope of the invention.

## Claims

1. An HSL protein, wherein having an amino acid sequence selected from at least one of the following:
(1) a first amino acid sequence derived from the mutation of at least one of the sites 140, 204, 298 and 335 in the amino acid sequence shown in SEQ ID NO: 1;
(2) a protein derived from the first amino acid sequence by substituting, deleting or adding one or at least two amino acids in the first amino acid sequence, or a protein represented by an amino acid sequence with a tag connected to the amino terminal and/or the carboxyl terminal of the first amino acid sequence.

2. The HSL protein as claimed in claim 1, wherein the first amino acid sequence is an amino acid sequence selected from at least one of the following:
(1a) an amino acid sequence derived from where the isoleucine at site 335 is mutated into phenylalanine shown in SEQ ID NO.1;
(1b) an amino acid sequence derived from where the phenylalanine at site 140 is mutated into histidine, the leucine at site 204 is mutated into phenylalanine, and the isoleucine at site 335 is mutated into phenylalanine shown in SEQ ID NO: 1;
(1c) an amino acid sequence derived from where the leucine at site 204 is mutated into phenylalanine, the phenylalanine at site 298 is mutated into leucine and the isoleucine at site 335 is mutated into phenylalanine shown in SEQ ID NO.1;
(1d) an amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into alanine shown in SEQ ID NO.1;
(1e) an amino acid sequence derived from where the phenylalanine at site 140 is mutated into histidine, the leucine at site 204 is mutated into phenylalanine, the phenylalanine at site 298 is mutated into leucine and the isoleucine at site 335 is mutated into tryptophan shown in SEQ ID NO.1.

3. The HSL protein as claimed in claim 1 or 2, wherein the first amino acid sequence is at least one of the amino acid sequences shown in I335F, F140H/L204F/I335F, L204F/F298L/1335F, F140H/L204F/F298L/I335A, F140H/L204F/F298L/I335W.

4. A gene encoding an HSL protein, wherein a nucleotide sequence of the gene is a nucleotide sequence capable of encoding the amino acid sequence of the HSL protein as claimed in any one of claims 1-3.

5. The gene as claimed in claim 4, wherein a nucleotide sequence of the gene is a nucleotide sequence selected from at least one of the following:
(1) a nucleotide sequence shown as SEQ ID NO. 11;
(2) a nucleotide sequence having at least 90% identity, preferably at least 91% identity, at least 92% identity, at least 93% identity, at least 94% identity, at least 95% identity, at least 96% identity, at least 97% identity, at least 98% identity, or at least 99% identity to SEQ ID NO. 11.

6. A recombinant vector, wherein the recombinant vector comprising the gene as claimed in claim 4 or 5.

7. A transgenic cell, wherein the transgenic cell comprising the gene as claimed in claim 4 or 5;
preferably, the transgenic cell is a prokaryotic cell.

8. A composition , wherein the composition comprising an HSL protein as claimed in any one of claims 1-3.

9. Use of at least one of the HSL protein as claimed in any one of claims 1-3, the gene as claimed in claim 4 or 5, the recombinant vector as claimed in claim 6, the transgenic cell as claimed in claim 7 in improving the herbicide resistance of crops.

10. Use as claimed in claim 9, wherein the herbicide is an HPPD inhibitor;
preferably, the herbicide is at least one of a triketone compound, a pyrazole compound, an isoxazole compound, a diketone nitrile compound and a heterocyclic amide compound;
more preferably, the herbicide is at least one of mesotrione, tembotrione, tefuryltrione, benzobicyclon technical,benquitrione, methyl-benquitrione, compound A, pyraquinate technical and topramezone.

11. A method for improving the herbicide resistance of crops, wherein the method comprises: transferring the recombinant vector as claimed in claim 6 into a target plant, so that the target plant expresses the HSL protein as claimed in any one of claims 1-3 to obtain herbicide resistance.

12. A method for improving the herbicide resistance of crops, wherein the method comprises: transferring the recombinant vector in the mutant crops containing the recombinant vector as claimed in claim 6 into a target plant through hybridization, transfer or backcross, so that the target plant expresses the HSL protein as claimed in any one of claims 1-3 to obtain herbicide resistance.

13. A method for improving the herbicide resistance of crops, wherein the method comprises: transforming the HSL gene of a target plant by CRISPR/Cas gene editing method, so that the target plant expresses HSL protein as claimed in any one of claims 1-3 to obtain herbicide resistance.
